# EUROPEAN PATENT SPECIFICATION

(11) **EP 1 238 641 B1**
(45) Date of publication and mention of the grant of the patent: **24.11.2004**
(21) Application number: 02013409.4
(22) Date of filing: 03.06.1996
(51) Int. Cl.: A61F 11/06, A61F 11/14

(54) **A covering device and an ear warmer**
Ohrenschützer und -wärmer
Protecteur et réchauffe-oreilles

(30) Priority: 02.06.1995 US 460587
(43) Date of publication of application: 11.09.2002
(62) Divisional of application: 96304006.8
(73) Proprietor: 180s, Inc., Baltimore Maryland 21202-3101 (US)
(72) Inventor: Legette, Brian E., Maryland 21224-3654 (US); Wilson II, Ronald L., West Virginia 26105 (US)
(74) Representative: Grünecker, Kinkeldey, Stockmair & Schwanhäusser Anwaltssozietät

(56) References cited:
- CH-A- 294 003
- FR-A- 2 536 253
- GB-A- 1 327 614
- US-A- 1 628 483
- US-A- 2 314 782
- US-A- 2 615 169
- US-A- 5 257 420

## Description

### Field of the Invention

The present invention relates to a covering to be worn over and to protect the ears of an individual. The covering is intended to extend around the back of the head or neck of the individual when being worn. The present invention also relates to an ear protection device that may incorporate audio earphones or similar type elements.

### Background of the Invention

There are a number of prior devices which cover an individual's ears for warmth or the like. These devices, generally known as "ear muffs", generally include a band which wraps around the top of the head and includes an enlarged end which engages and covers the ears for warmth and protection.

FR 2536253 discloses an ear muff including a flexible band comprised of two portions that are movable relative to each other so as to adjust the relative length of the flexible band, wherein a desired length may discontinuously be adjusted by a plurality of studs provided in one of the two portions, thereby fixing the relative position. This document does not disclose first and second curved portions of a band that has an enlarged portion at one end and a passageway at the other end and a second slot portion that allows the enlarged portion of the opposite curved portion of the band to slide through.

Despite the existence of various types of ear muffs and headbands, there remains a need for an ear protection device having the advantages of the present invention.

### Summary of the Invention

The present invention relates to an ear warmer frame to be worn over the ears of an individual. Preferably, the ear warmer frame extends between the opposite ears around the back of the individual's head or neck.

According to the present invention, an ear warmer frame as defined in claim 1 is provided.

### Brief Description of the Drawings

For the purpose of illustrating the invention, there is shown in the drawings a form which is presently preferred; it being understood, however, that this invention is not limited to the precise arrangements and instrumentalities shown.
Figure 1 shows one form of the covering device usable with the present invention as being worn by an individual.
Figure 2 shows a top elevational view of an ear covering device.
Figure 3 shows a side plan view of the frame of a covering device.
Figure 4 shows a front plan view of the frame of a covering device.
Figure 5 shows a bottom elevational view of the frame of a covering device.
Figure 6 shows the covering device incorporating a headphone type speaker arrangement therein.
Figures 7A and 7B show the component parts of the covering device and headphone speakers as contemplated by Figure 6.
Figures 8A through 8F show a series of covering panels which form the fabric covering for the present invention.
Figures 9 through 15 show various embodiments of a hinged connection for use with the ear covering device of the present invention.
Figures 16 through 24 show side elevational views of various embodiments of a "living hinge" for use within the covering device of present invention.
Figure 25 shows a collapsible frame for an ear covering device.
Figure 26 shows the collapsible frame of Figure 25 illustrated in an opened position.
Figure 27 shows an ear covering device in accordance with the present invendion, illustrated in the collapsed position.
Figure 28 is a top plane view of a portion of an alternate embodiment of an ear covering device.
Figure 29 is a side elevation of the alternate embodiment of the ear covering device as shown in Figure 28.
Figure 30 shows a side elevation of the ear warmer frame of the present invention.

### Detailed Description of the Invention

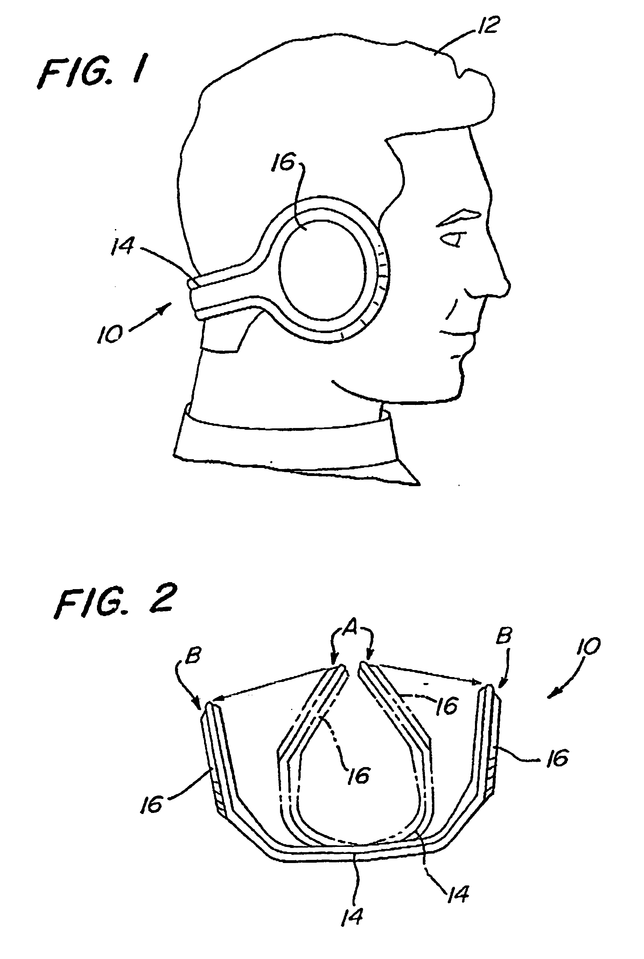

In the Figures, where like numerals indicate like elements, there is shown multiple forms of an ear covering device. The ear covering device as illustrated in Figure 1 is generally designated by the numeral 10. The ear covering device 10 is adapted to be worn over the ears of an individual 12. When worn, the device 10 extends around the back of the head and/or neck of the wearer.

The ear covering device 10 generally includes a band portion 14 and two ear protecting portions 16. One side of the ear covering device 10 is illustrated in Figure 1. The opposite side of device 10 is contemplated to be a mirror image of that illustrated in Figure 1. As illustrated in Figure 1, the band portion 14 is relatively narrower than the ear covering portions 16. It is contemplated that variations on this dimensional relationship may be made without departing from the essence of the present invention.

Figure 2 shows a top plan view of the ear covering device 10 illustrated in Figure 1. In position "A" in Figure 2, the ear covering device 10 is illustrated in its normal rest position with the ear covering portions 16 positioned closely adjacent to one another. The rest positioning of the covering portions is created by the curvature of band portion 14 and the form of the frame for the ear covering portions 16 (shown in detail in other figures). Position "B", as illustrated in Figure 2, shows the ear covering portions 16 deflected outwardly due primarily to a flexing of band 14. The enlargement of the distance between opposite ear covering portions 16 permits the ear covering device 10 to be placed on the head of the individual 12, as illustrated in Figure 1. The spring force created by the flexible band 14 of the ear covering device 10 causes the ear covering portions 16 to engage against the side of the head of the individual 12. The spring force of the band is contemplated to be sufficient to engage the head 12 so that the ear covering device 10 does not fall off. However, the spring force is desired to be limited so that the ear covering device is not uncomfortable to wear.

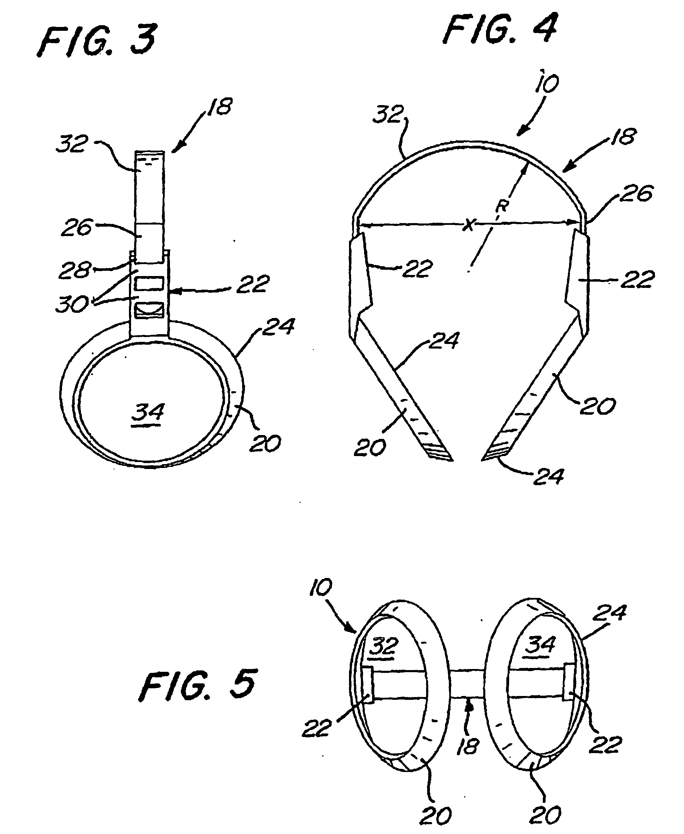

Figures 3-5 generally illustrate the framework of covering device 10. The frame generally comprises a flexible band 18 and two ear cup frame members 20. The ear cup frame members 20 include an attachment portion or flange 22 and a frusto conical frame portion 24. The attachment portion 22 in the embodiment illustrated in Figures 3-5 is integrally formed with the frusto conical portion 24. The attachment portion 22 is formed at an oblique angle with respect to the plane of the frusto conical portion 24. This angle creates, in part, the inward positioning of the ear covering portions 16 as illustrated in Figure 2, i.e., position "A". The angle between the attachment flange 22 and the frusto conical portion 24 is contemplated to be in the range of about 90 to 160° and is preferably approximately 145°. The flexible band 18 includes end portions 26 which are engaged within the attachment portions 22 of the ear cup frame members 20. As illustrated in Figure 3, the end portion 26 is engaged within a slot 28 within attachment portion 22 and is secured therein by ribs 30. A detent (not shown) may be provided on the end portion 26 of the flexible band 18 so as to resist the removal of the ear cup frame member 20 from between the opposing ribs 30 on the end portion 26.

The flexible band 18 as illustrated includes a curved portion 32 and two end portions 26. Preferably, the curved portion 32 has a radius of curvature of approximately 5.72 cm (2 1/4 inches). The end portions are formed by bends at the end of the curved portion 32 and are separated by a distance X, as illustrated in Figure 4. Preferably, distance X is in the range of about 8.9 to 11 cm (3.5 to 4.5 inches) and specifically about 9.9 cm (3.9 inches). The end portions 26 are straight and are formed integral with the curved portion 32. Preferably, the band 18 is made of a stainless steel type material; however, other materials are contemplated, including polypropylene, nylon, polyethylene, etc. The band 18 should be sufficiently flexible such that the relative distance X between the two end portions 26 may be enlarged and the ear cup frame members 20 may be separated prior to placing ear covering device 10 on the individual 12 (Figure 1).

The ear cup frame members 20 are preferably made of a plastic material, such as nylon. The frusto conical portions 24 at each end of the frame include a central opening 34. The diameter of the frusto conical portion at its base is contemplated to be in the range of about 6.9 to 10 cm (2.7 to 4 inches) and preferably in the neighborhood of 8 cm (3 inches). The preferred height of the frusto conical portion is in the range of about 0.5 to 1.3 cm (0.2 to 0.5 inches). The preferred height dimension if approximately 0.64 cm (0.25 inches) with the side wall being at an angle of about 45°. The frusto conical portion 24 is contemplated to fit around the ear when wearing of the ear covering device 10.

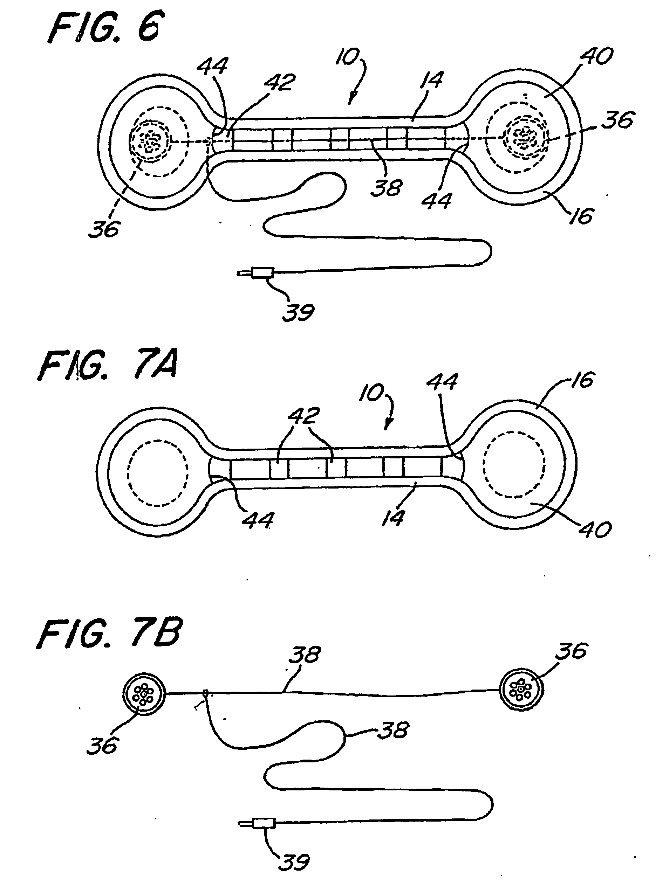

In Figure 6, the ear covering device 10 is shown having a pair of speakers or earphones 36 and a speaker wire 38 incorporated therein. The earphones 36 and speaker wire 38 are also illustrated in Figure 7B, separate from the ear covering device 10. The speaker wire 38 terminates in a standard male jack 39 for connection to a personal radio, tape player or the like (not shown).

In Figures 6 and 7A, the ear covering device 10 includes band 18 and ear cup frame members 20 as shown in Figures 3-5. The band 18 and ear cups 20 are covered by a fabric material 40. As illustrated, the band portion 14 and the ear covering portions 16 are each covered with fabric 40. The fabric covering 40 includes a series of bands 42 which engage the speaker wire 38 and retain it within the ear covering device 10. In addition, a pocket or cavity is formed within the ear cups 20 which retains the earphones 36. The earphones 36 may be inserted into the pocket or cavity by means of slot 44 within the fabric 40. As illustrated, the slot 40 is positioned adjacent to each ear covering portion 16.

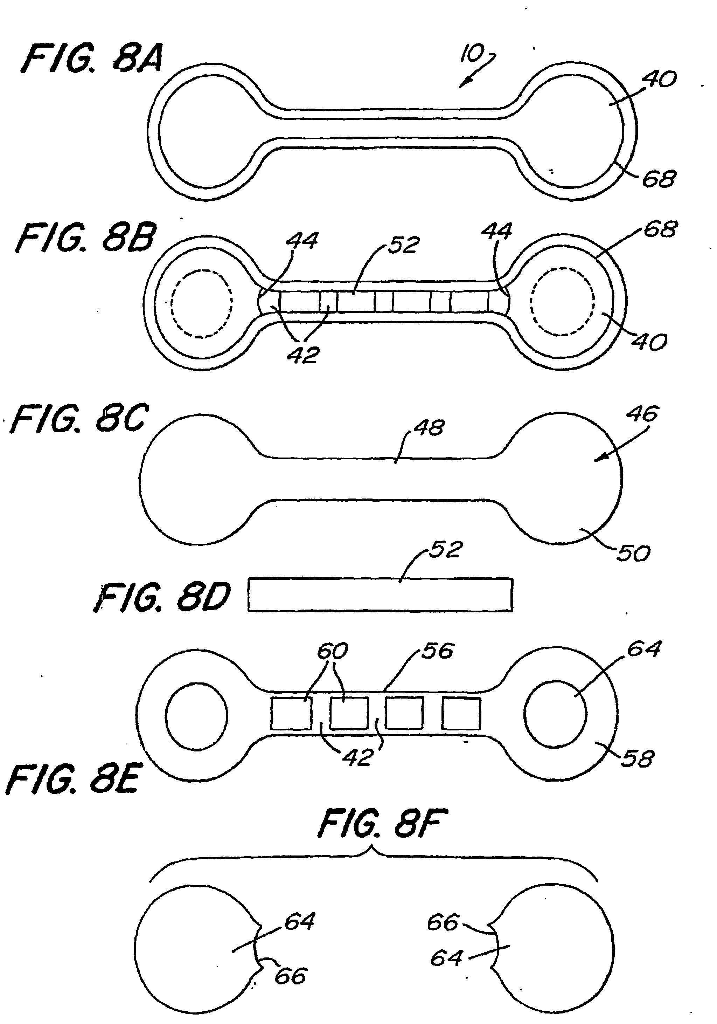

As generally illustrated in Figures 8A-8F, the fabric 40 includes multiple layers. In Figure 8A, there is shown a top plan view of the ear covering device 10 in the open position. The fabric 40 completely covers the outside surfaces of the band 18 and the ear cup frame members 20. As shown in Figure 8B, the fabric 40 also covers the inside surfaces of the band 18 and ear cups 20.

Multiple layers of fabric are utilized to form the fabric covering 40 for the ear covering device 10. These layers are generally illustrated in Figures 8C-8F. In Figure 8C, there is shown an outer shell 46 which includes a central tapered portion 48. In Figure 8D there is shown an inner fabric covering 52 which when assembled is exposed between bands 42, as shown in Figure 6. The inner fabric 52 generally covers the inner surfaces of band 18. The inner fabric 52 is overlapped by a central portion 56 which is shown in Figure 8E. The central portion 56 includes a series of alternating bands 42 and openings 60 in its center and two circular portions 58 at each end. A central opening 62 is provided within the circular portions 58. The central portion 56 is contemplated to be provided on the inside of the ear covering device 10 and at least partially covering the inner fabric 52. Covering the circular portions 58 of the band fabric 56 are inner ear members 64. The inner ear members 64 as shown in Figure 8F have a generally circular configuration. The inner ear members 64 are positioned over top of the circular portions 58 and the corresponding central opening 62 in central fabric 56. Adjacent one end of the inner ear members 64 is a curved border 66 which in the final constructions forms the slot 44 for insertion of the earphones 36 as contemplated by Figures 6 and 7.

The various layers of the fabric material 40 are combined by sewing or the like. The fabric covering may include an outer piping 68. Piping 68 may be formed as part of one of the layers of the fabric material 40 or may be a separate element attached thereto.

Any number of materials are contemplated for the fabric 40. Piping 68 as illustrated in Figures 8A and 8B may be a wear-resistant cotton blend or an entirely synthetic material. The exposed outer shell 46 may be a nylon-covered close cell neoprene or other moisture-resistant fabric. The band fabric 54 may also be made of neoprene. Inner central tapered fabric 52 and inner ear members 64 are preferably made of a synthetic fleece type lining material, so as to provide warmth and comfort. The inner ear members 64 will be in direct contact with the individual 12. It is noted that the exposed outer shell 46 may alternatively be made of a fleece material. It has been determined that the fleece material provides sufficient protection from the elements while also permitting sound to pass therethrough.

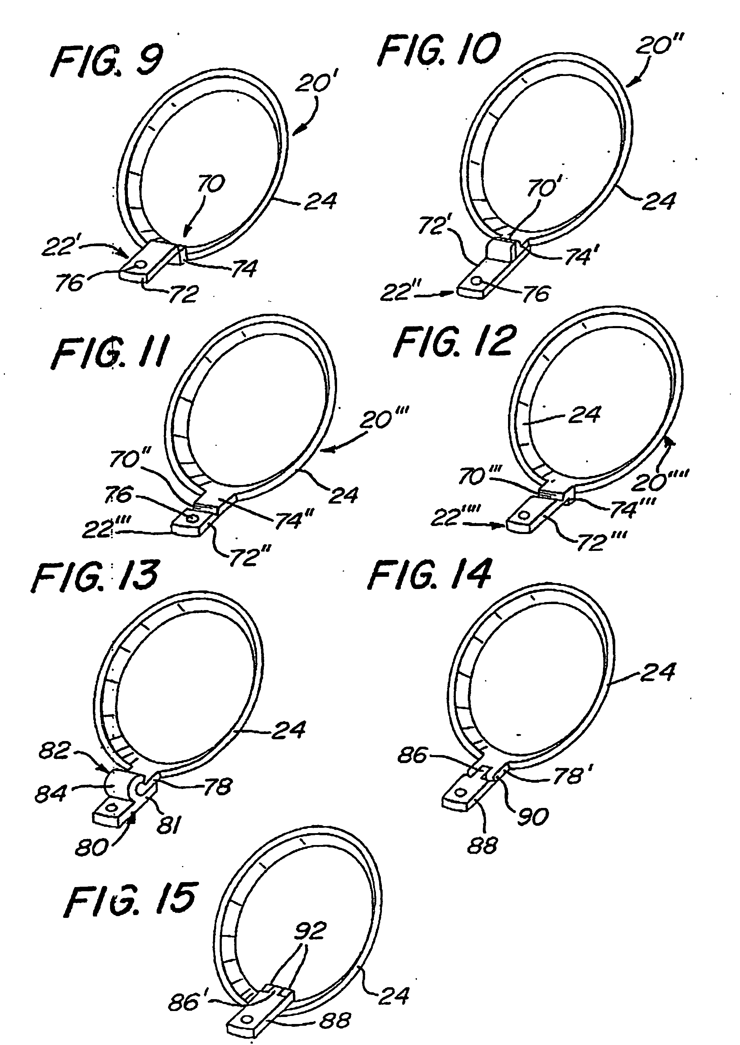

In Figures 9-23, there is illustrated a number of hinge embodiments which may be used to form the connection between the attachment portion 22 and the frusto conical portion 24 of the ear cup frame members 20. Each of these embodiments will be discussed separately below. A number of these embodiments include a living hinge type arrangement. A living hinge as contemplated by the present invention generally includes a continuous plastic formation having a reduced cross-section between a base flange and an attachment flange, wherein flexing is permitted between the two flanges. The form of each of the hinges will be discussed in further detail below.

In Figure 9, there is illustrated an embodiment of the attachment portion 22' for the ear cup frame member 20'. The frusto conical portion 24 as illustrated in this Figure 9 is generally contemplated to be the same as that illustrated previously. However, the communication between the frusto conical portion 24 and the attachment portion is not contemplated to be rigid. The living hinge 70 is provided between an attachment flange 72 and a base flange 74. The living hinge 70 permits the ear cup 20 to pivot such that the ear covering portion 16 may collapse into the band portion 14 to reduce the size of the ear covering device 10 when not in use. A rivet hole 76 is provided in the attachment flange 72. The engagement between engagement portion 22' and the flexible band 18 (not shown in Figure 9) may be made by means of a rivet (not shown). It is contemplated that rotation of the ear cup frame member 20' about the rivet or opening 76 further permits collapsing the ear covering portions 16 into band 14.

In Figure 10 there is illustrated a further embodiment of the ear cup frame member 20" which includes a frusto conical portion 24 and a modified living hinge 70'. The attachment portion 22" generally includes an inverted T-shaped attachment flange 72' and an L-sbaped base flange 74'. The connection between the T-shaped flange 72' and the L-shaped flange 74' forms the living hinge 70' and limits the amount of outward rotation of the frusto conical portion 24 with respect to the attachment portion 22". The hinge 70' permits the inward rotation of the frusto conical portion 24 towards the attachment portion 22" when collapsing the ear covering device 10. Again, a rivet hole 76 is provided in the attachment flange 72' for securing the attachment portion 22" to the end portion of the flexible band 18 (above).

In Figure 11, there is shown a further embodiment of an ear cup frame member 20'". In this embodiment, the attachment flange 72" is generally planar with the base flange 74'' and includes the hinge 70'' positioned therebetween. A slot or groove is provided as part of the living hinge 70'' such that a limited amount of inward rotation of the frusto conical portion 24 is permitted with respect to the attachment flange 72''. Again, the attachment flange 72" may be secured to band 18 (above) by means of rivet hole 76 and a rivet (not shown).

In Figure 12 there is illustrated a still further embodiment of an ear cup frame member 20"" including a hinge 70"' formed as part of the attachment portion 22"". The base flange 74''' in this embodiment generally includes an L-shaped member with one leg of the L-shape being generally planar with the attachment flange 72'''. The living hinge 70"' is provided between the L-shaped flange 74"' and the attachment flange 72"' with the projection of L-shaped flange 74''' serving to prevent full rotation of the frusto conical portion 24 with respect to the attachment flange 72'''.

In Figures 13-15, there are illustrated further hinge embodiments for the ear cup frame members. These hinged arrangements are generally contemplated to include a press fit relationship and/or a pin secured within adjacent elements.

In Figure 13, the frusto conical portion 24 includes a projecting tab 78. Tab 78 includes a rounded end and forms the axis for the hinge 82. Tab 78 is formed integral with the frusto conical portion 24. The securing portion 80 of hinge 82 includes a planar base 81 and a curved projection 84. The curved projection 84 generally encapsulates the end of the projecting tab 78 such as a hinge is formed. The planar base 81 of the securing portion 80 generally limits outward rotation of the frusto conical portion 24. The curve portion 84 may also limit inward rotation about the hinge 82.

In Figure 14, the frusto conical portion generally includes a projecting tab 78' which includes a central slot. A tab 86 on the securing portion 88 is engaged within the slot in tab 78. A pin 90 forms an axis for the hinge and extends through the ends of tabs 78 and 86.

In Figure 15 there is shown a further embodiment of a hinge construction for the ear cup frame. The frusto conical portion 24 includes two projections 92 which are formed on the inside surface of the cone 24. The base members 92 engage on opposite sides of a projecting tab 86' which is formed as part of the securing portion 88. A pin (not shown) forms the axis for the hinge and extends through the projections 92 and the tab 86' on securing portion 88.

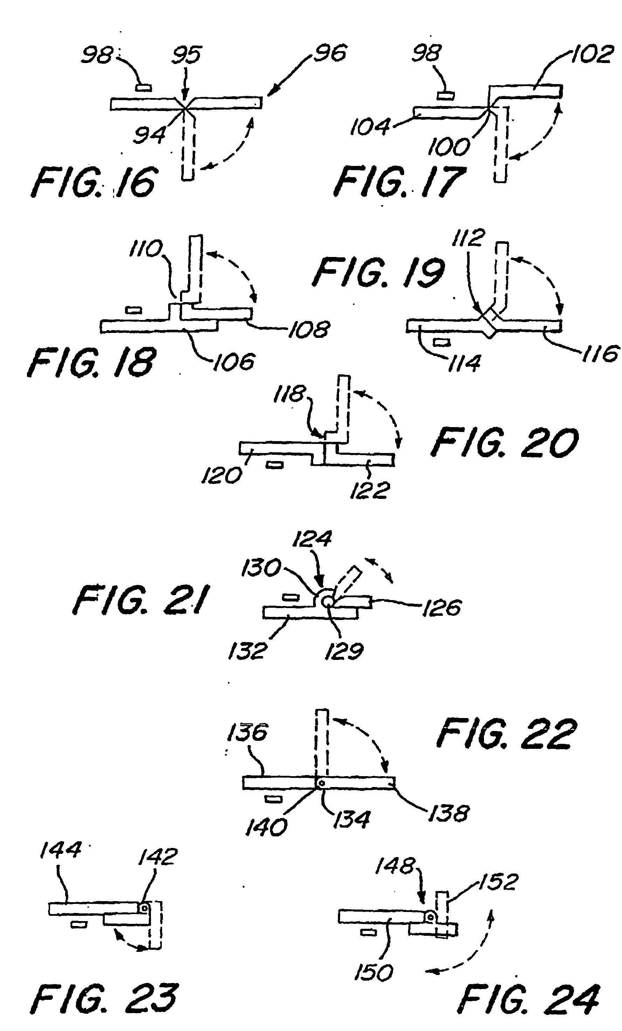

In Figure 16 there is illustrated a living hinge in the form similar to that shown in Figure 11. The hinge 94 generally includes a V-shaped notch 95 within a planar body portion 96. Projecting from the left hand element of the planar body portion 96 is an attachment head 98 which may be inserted into an opening in the end portion 26 of band 18 (not shown). It is contemplated that the press fit relationship between the opening in end portion 26 and the attachement head 98 will form a pivot similar to the rivet attachment contemplated for the embodiments in Figures 9-15. In phantom, there is illustrated the flexing of the living hinge 94.

In Figure 17, a further embodiment of the living hinge is shown. The hinge 100 is formed between a first base member 102 and a second base member 104. The second base member 104 includes an attachment head 98, similar to that contemplated by Figure 16. However, in this embodiment, the first base member is not planar with the second base member 104. The flexing of the hinge 100 is shown in phantom.

In Figure 18, there is illustrated a living hinge having a form similar to that shown in Figure 10 above. A first base member 106 forms an inverted T-shaped element and is provided along with an L-shaped base member 108. The hinge 110 is formed between the first and second base numbers 106, 108. Because of the projecting portion on the right hand side of the first base member 106 (as illustrated in Figure 18) the downward rotation of the second base member 108 is limited. The movement of the second base member 108 is shown in phantom.

In Figure 19, there is a shown a still further embodiment of a living hinge type attachment for the ear cup member. The hinge 112 is formed between a first base member 114 and a second base member 116. The first and second base members 114, 116 each include a bend at their respective ends which permit the formation of the hinge 112. The bent ends engage one another so as to limit rotation of second base member 116 in the downward direction (as shown). The rotation of member 116 in the upward direction is shown in phantom.

In Figure 20, there is a further embodiment of a living hinge type structure. In this embodiment, the hinge 118 is formed between two L-shaped members 120 and 122. The L-shaped members are positioned opposite one another, such that their elongated projections are not planar. This arrangement again limits downward rotation of the right hand L-shaped member 122. The upward rotation of member 122 is shown in phantom in this Figure 20.

In Figure 21, there is illustrated a hinge arrangement similar to that contemplated by Figure 13 above. The hinge 124 is formed by a tab 126 having a circular end 128 which is engaged within a curved projection 130 formed on a planar member 132. The tab 126 engages the upward surface of planar 132 so as to limit rotation thereof downwardly about the hinge 124. In addition, the upward rotation of tab 126 about the hinge 124 is limited as illustrated in phantom. The upper surface of tab 126 engages the end the of curved portion 130. The hinge 124 is contemplated to be formed by a press fit relationship between the rounded end 128 of tab 126 and the inside surfaces of curved projection 130.

In Figure 22, there is illustrated a further embodiment of a hinge which is generally similar to that shown in Figure 14. The hinge 134 is formed between a first member 136 and a second member 138. It is contemplated that the first and second member include engaging projections which are formed around a central pin 140. The upward rotation of second member 138 about the hinge 134 is shown in phantom.

In Figure 23, there is illustrated a further embodiment of a hinge connection. The hinge 142 is formed between a first planar member 144 and an L-shaped second member 146. The L-shaped second member 146 permits the upper surface of the second member 146 to engage the lower surface of the first planar member 144 when the binge 142 is collapsed. The opening of hinge 142 is shown in phantom.

In Figure 24, there is illustrated a further hinge embodiment. The hinge 148 is formed between a first planar member 150 and a T-shaped second member 152. The hinge is formed on the central portion of the T-shape in a manner similar to that contemplated by the hinge 134 in Figure 22 and the hinge 142 in Figure 23. Again, the upper portion of the T-shaped member 152, when in inverted position, engages the lower surface of the planar member 150 to limit rotation about the hinge 148. The rotation of hinge 150 is illustrated in phantom.

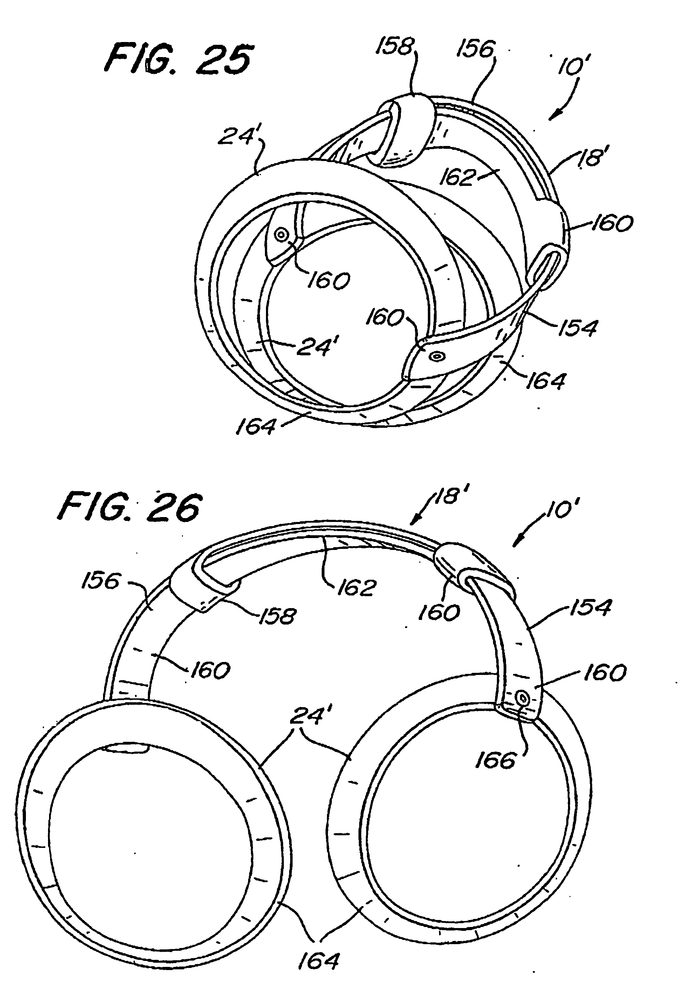

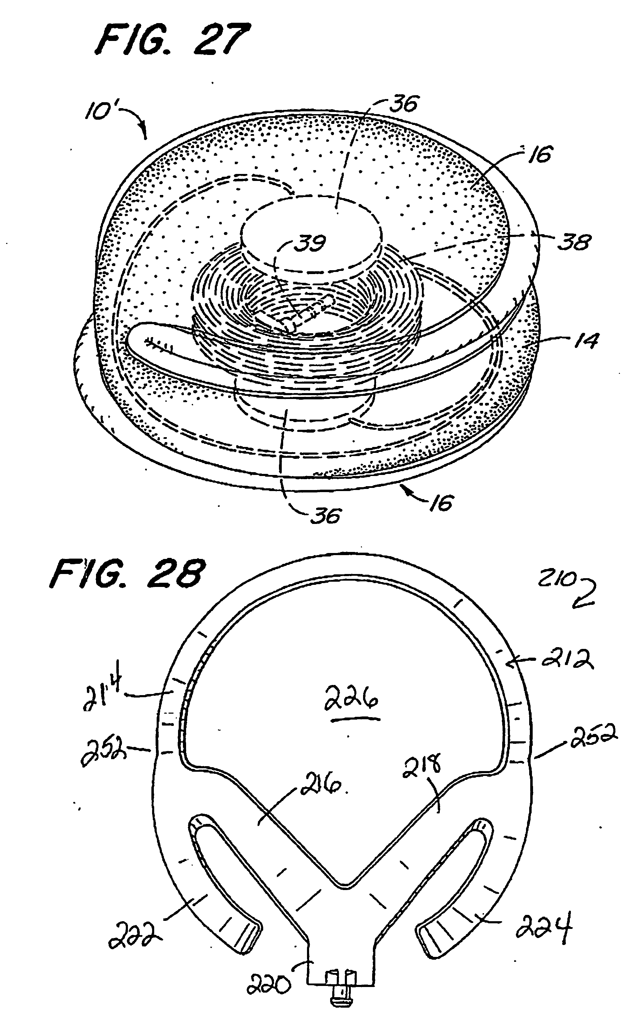

In Figures 25, 26 and 27, there is illustrated a collapsible ear protection device 10' which is shown in the open position (Figure 26) and in the closed position (Figures 25 and 27). For illustrative purposes, only the frame portion of the ear protection device 10' has been shown in Figures 25 and 26. However, a fabric covering similar to the embodiment shown in the figures discussed above is contemplated to be included and is shown in Figure 27.

The band 18' of device 10' generally includes a semi-circular first portion 154 and a semi-circular second portion 156 which engage one another to form a continuous arc. Engagement is formed by wrapping a portion of each member 156 and 154 around the other. The wrapping portion of the first semi-ciccular portion 154 is identified by the numeral 158. The wrapping portion of the second semi-circular member 156 is identified by the numeral 159. As an alternative to wrapping the two portions around one another, separate rings or bands may be wrapped around one end of the first member 154 (for example) and attached thereto. The rings will also be wrapped around the central portion of the second member 156. In final assembly, as illustrated in Figure 26, the end of the band 18', identified by the numeral 160, includes a single layer whereas the central portion 162 of the band 18' includes an overlapped double layer. The double layer portion may be shortened or elongated by sliding the two members 154 and 156 with respect to one another so as to move the wrapping portions 158 and 159 closer together. To shorten the overall length of the band 18', the two wrap portions 158 and 159 are moved further apart from one another (elongating the overlap 162 of the two portions, but shortening the overall length of the band 18').

In Figures 25 and 26, the frusto conical portion 24' forms the entirety of the ear cup 164. The connection between the ends 160 of the band 18' is preferably formed by an attachment head 166, similar to that specifically illustrated in Figure 16 (element 98; also illustrated in Figures 19-22), and a socket or opening in the ends 160 of the band 18'. As illustrated, the attachment bead is inserted in the opening in a press fit type relationship. Alternatively, the ends 160 could include an inverted V-shaped notch with a rounded opening at the top. The shaft of the attachment head in this contemplated embodiment would be slid into the notch and retained within the rounded opening, again in a press fit type relationship. Another possibility is a "figure 8" type opening with one side thereof being larger than the other. In this form, the enlarged diameter of the head portion can be inserted into the larger opening and then the shaft portion slid into the smaller portion of the opening (again in a press fit relationship). Other forms of detachable fastening and non-detachable fastening (such as a rivet) are contemplated. The intent of this type attachment is to permit the frusto conical portion 24' to rotate about the attachment head 166 and the end of the band 18'.

As is more particularly illustrated in Figure 27, one frusto conical portion 24' formed within the ear covering portion 16' may be rotated so as to flatten against the opposite ear covering portion 16' (which is also rotated) with the central portion 14 (which covers band 18') forming a circular (or at least semi-circular) side wall between the parallel ear covering portions. In this manner, the collapsed ear covering device 10' can retain the speaker wire 38 and male jack 39 (each shown in phantom). The speakers 36 (also shown in phantom) are retained within the ear covering device within the pocket or cavity formed by the central opening 62 within the central fabric portion (Figure 8E) and the respective outer shell 46 (Figure 8C) and inner ear members 64 (Figure 8F).

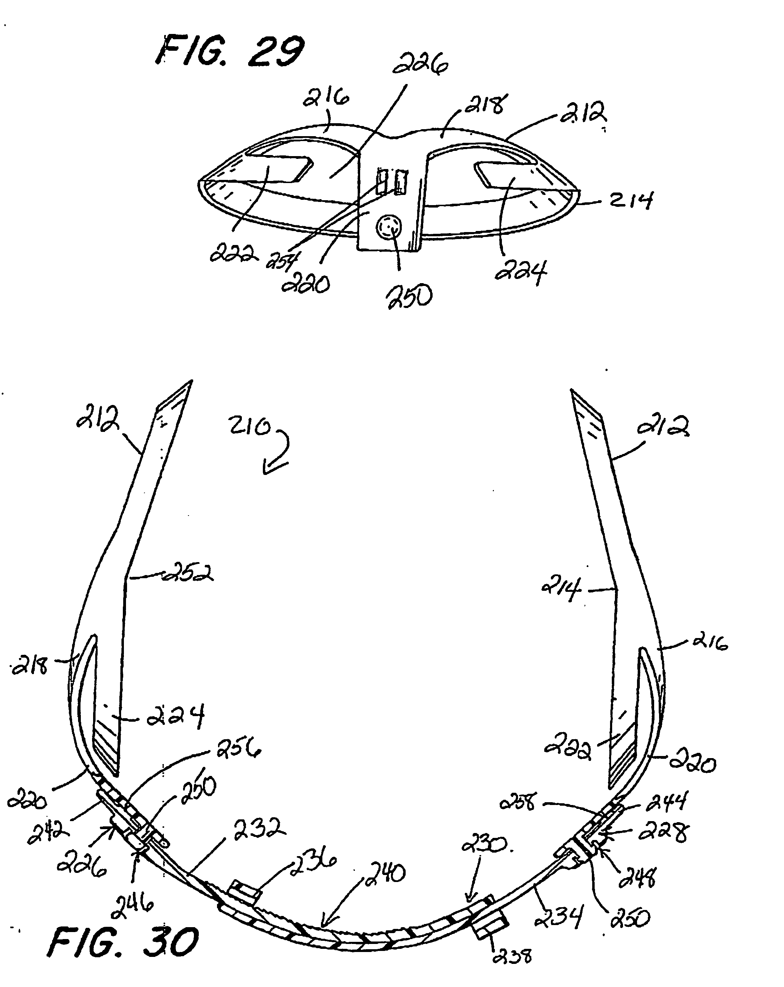

In Figures 28-30, there is shown an alternate embodiment of a frame for an ear protection device. The frame of this embodiment is generally labeled with the numeral 210. In Figures 28 and 29, there is particularly illustrated an ear cup frame member 212. The ear cup frame 212 includes a modified ear cavity having a semi-circular frame 214 at one end, two support flanges 216, 218 formed as a central V-shaped portion, an attachment portion or flange 220, and two cup extension portions 222 and 224. The modified ear cavity is generally shown in Figure 28. The semi-circular portion 214 defines, along with the support flanges 216, 218, a central opening 226. The attachment flange 220 extends outwardly from the vertex of the V-shape of the support flanges 216, 218.

As illustrated more particularly in Figures 29 and 30, the outline of the cavity of ear cup 212 is generally formed by the cup extensions 222, 224, along with the semi-circular frame portion 212. The support flanges 216, 218 project above the ear cup to further define the cavity for receiving an ear. Thus, the frame member 212 forms a open area defined by the height of the semi-circular portion 214 and the cup extensions 222 and 224, as well as the projection of the support flanges 216, 218 thereabove.

In Figure 30, there is shown the ear warmer frame according to the present invention. As shown in Figure 30, the frame 210 includes a band 230 which is comprised of a first curved portion 232 and a second curved portion 234. The two band portions 232, 234 overlap and engage one another by means of passageways 236, 238. Passageway 236 is positioned at one end of band portion 234, which is opposite band end 228. Passageway 238, formed as part of the band portion 232, is positioned at the opposite end of band 234 from attachment end 226. Band portion 232 on its inside surface includes a series of raised bumps or ratchet teeth 240 which form a resistance to the sliding movement of passageway 236 of band portion 234 when the expansion of the overall length of the band 230 is desired. The passageways 236 and 238 include a lower portion in which the opposing band slides. As can be seen, the thickness of the band ends 226, 228 increases in the area of the openings 246, 248. An upper portion is included in the passageways which permits the passage of the passageway 236 over the end 226 of band portion 232 and, similarly, the passage of passageway 238 over the band end 228 of band portion 234. Each band end 226 and 228 includes a projecting tab 242 and 244, respectively, which extends beyond the openings 246 and 248, respectively, which receive the attachment head 250 of the two ear cup frame members 212.

The connection between the attachment head 250 and the openings 246 and 248 in the band portions 232 and 234, respectively, permits the rotation of the ear cup around the axis of the attachment head 250. When the band ends 226, 228 are aligned with the attachment flange 220, the tabs 242, 244 form a moment arm which translates the spring force, created by the curvature of the band 230, to the ear cup frame members. This permits the ear cup frame member to flex and engage around the ear of the wearer. The semi-circular portion 214 flexes with respect to the more rigid structure of the support flanges 216, 218 due to the "twist" line created by the relief area 252 on opposite sides of the ear cup frame 212. In addition, the attachment flange 220 is provided with raised bumps 254 on the outside surface thereof. The raised bumps define a slot therebetween in which is engaged a bump 256 on the inside surface of band end 242 of band portion 232 or a bump 258 on end 228 of band portion 234. The engagement of bumps 256, 258 within the slot formed by the raised portions 254 on attachment flange 220 secures the ear cup frame member 212 in alignment with band 230. This engagement can be overcome by a slight rotation of the ear cup 212 with respect to the band 230.

The overall structure of the embodiment shown in Figures 28-30 is contemplated to be generally larger than those shown in the earlier embodiments. In addition, the cavity of the ear cup frame member 212 is contemplated to have a more semi-circular interior due to the inclusion of the support flanges 216 and 218 and due to their relationship with the attachment flange 220.

The frame embodiment 210 shown in Figures 28-30 is contemplated to include a fabric covering similar to those discussed above. In addition, speakers, such as those shown in Figures 6, 7b and 27, may be included within the interior of the ear covering device 210.

The frame of this or any embodiment may be made of a Crastin® material which is manufactured by the DuPont Company. The Crastin® material is considered less suspectable to changes in moisture than a typical nylon material and is not severely effected by changes in temperature. It is contemplated that the characteristics of this material and its higher flex modulus will enable the frame to be relatively thinner than would be possible by a the use of nylon material, while still accomplishing the desired characteristics of the present invention.

## Claims

1. An ear warmer frame (210), comprising:
a band (230) having:
a first curved portion (232) having an enlarged portion (246) at a first end (226) and a passageway (238) at a second end, and
a second curved portion(234) having an enlarged portion (248) at a first end (228) and a passageway (236) at a second end, each passageway (236,238) having a first slot portion allowing the opposite curved portion of said band (230) to slide through and having a second slot portion allowing the enlarged portion (246,248) of the opposite curved portion of said band to slide through, whereby one end of each of the first and second curved portions (232,234) overlapping and slidably attached to opposing curved portion, such that the relative overall length of the band (230) may be adjusted.

2. The ear warmer frame of claim 1, further comprising:
two ear cups each having an attachment head, each attachment head attaching to one enlarged portion of one curved portion of said band.

3. The ear warmer frame of any one of claim I or 2, wherein the passageway of the first curved portion of said band is integrally formed with the first curved portion of said band, the passageway of the second curved portion of said band is integrally formed with the second curved portion of said band.

4. The ear warmer frame of any one of claims 1 to 3, wherein the first curved portion of the band includes a series of raised bumps on the interior surface thereof which engage the ovrlap formed by the second band portion to form a resistance to the relative sliding movement of the two band portions when adjustment of the overall length of the band is desired.

5. The ear warmer frame of claim 1, adapted to be worn over the ears of an individual and extending around the back of the individual's head or neck, comprising:
two ear cups (20), each ear cup attached to the opposite end portions of the band, the attachment being formed by an attachment flange (22), each ear cup (20) having a cup portion (24) having a central opening (34) therein, the curvature of a central portion (32) of the band and the attachment flange (22) with the cup portion (24) directing the ear cups (20) inwardly towards one another from the opposite ends of the band (18); and
fabric means (40) covering the band (18) and the ear cups (20) on both sides.

6. The ear warmer frame as claimed in claim 5, wherein the cup portion has a frusto conical shape.

7. The ear warmer frame as claimed in any one of claims 5 or 6, wherein the attachment flange is integrally formed with the cup portion at an oblique angle thereto.

8. The ear warmer frame as claimed in any one of claims 5 to 7, wherein the fabric means forms a pocket within the cup portion of the ear cups.

9. The ear warmer frame device according to claim 5 wherein
said two ear cups are attached to the ends of the first curved portion and the second curved portion opposite their overlap, respectively, the attachment of the ear cup to the band permits rotation of the cup portion about the attachment.

10. The ear warmer frame as claimed in claim 9, wherein the fabric means envelopes the band and the ear cups.

11. The ear warmer frame as claimed in any one of claims 9 or 10, wherein the cup portion has a frusto conical shape.

12. The ear warmer frame according to claim 5, wherein
said two ear cups are attached to the free end of the first curved portion and to the free end of the second curved portion, respectively, the ear cups defining a cavity formed from two support flanges, and a semi-circular frame portion which extends between opposite projections of the support flanges, the semi-circular portion and the support flanges defining a central opening, the attachment flange projecting from the support flanges in a direction opposite the semi-circular portion, the attachment flange including means thereon for rotatably attaching the ear cups to the free ends of the first and second curved portions.

13. The ear warmer frame as claimed in claim 12, wherein said two support flanges are formed in a V-shape, and the attachment flange projects from the vertex of the support flanges.

14. The ear warmer frame as claimed in any one of claims 12 or 13, wherein the fabric means forms a pocket within the cavity of the ear cups.

15. The ear warmer frame as claimed in any one of claims 12 to 14, wherein the fabric means is constructed from a plurality of fabric pieces.

16. The ear warmer frame as claimed in any one of claims 12 to 15, wherein the two ear cups rotatably engage into a position at the free ends of the first and second curved portions so that the ear cups are aligned with the band.

17. The ear warmer frame as claimed in any one of claims 12 to 16, wherein:
a section adjacent to the end of the first curved portion of the band is less wide than the end of the first curved portion of the band, and
a second adjacent to the end of the second curved portion of the band is less wide than the end of the second curved portion of the band.

18. The ear warmer frame as claimed in any one of claims 12 to 17, wherein the band and the two support flanges direct the ear cups inwardly towards one another to maintain position of the covering device.

19. The ear warmer frame as claimed in any one of claims 12 to 18, wherein the covering device retains the position around the back of the individual's head or neck without any support around the top of the individual's head.

20. The ear warmer frame according to claim 5, wherein said band is flexible and at least a portion of said ear cup has a frusto conical configuration and a central opening.

21. The ear warmer frame of claim 20, wherein the fabric means and the ear cups form a pocket, whereby opening means are formed in the fabric means for insertion of an earphone into the pocket.

22. The ear warmer frame of any one of claims 20 or 21, wherein the flexible band has an inner side and an outer side, each ear cup has an inner side and an outer side, and the fabric means covers both the inner side and the outer side of the flexible band and covers both the inner side and the outer side of each ear cup.

23. The ear warmer frame of one of clams 20 to 23, wherein the ear cups are hingedly connected to the end portions of the band.

24. The ear warmer frame of claim 23, wherein the hinge connection between the end portions and the band is formed by a living hinge.

## Patentansprüche

1. Ohrenwärmerrahmen (210) mit:
einem Band (230), das aufweist:
einen ersten gekrümmten Bereich (232) mit einem vergrößerten Bereich (246) an einem ersten Ende (226) und einer Durchführung (238) an einem zweiten Ende, und
einen zweiten gekrümmten Bereich (234) mit einem vergrößerten Bereich (248) an einem ersten Ende (228) und einer Durchführung (236) an einem zweiten Ende;
wobei jede Durchführung (236, 238) einen ersten Schlitzbereich, der ein Durchgleiten des gegenüberliegenden gekrümmten Bereichs des Bandes (230) ermöglicht, und einen zweiten Schlitzbereich aufweist, der ein Durchgleiten des vergrößerten Bereichs (246, 248) des gegenüberliegenden gekrümmten Bereichs des Bandes ermöglicht,
wodurch ein Ende jeweils des ersten und des zweiten gekrümmten Bereichs (232, 234) überlappen und verschiebbar an dem gegenüberliegenden gekrümmten Bereich so angebracht sind, dass die relative Gesamtlänge des Bandes (230) einstellbar ist.

2. Ohrenwärmerrahmen nach Anspruch 1, der ferner umfasst:
zwei Ohrmuschelaufnahmen mit jeweils einem Befestigungskopf, wobei jeder Befestigungskopf an einem vergrößerten Bereich eines gekrümmten Bereichs des Bandes angebracht ist.

3. Ohrenwärmerrahmen nach Anspruch 1 oder 2, wobei die Durchführung des ersten gekrümmten Bereichs des Bandes und der erste gekrümmte Bereich des Bandes als eine Einheit ausgebildet sind und wobei die Durchführung des zweiten gekrümmten Bereichs des Bandes und der zweite gekrümmte Bereich des Bandes als eine Einheit ausgebildet sind.

4. Ohrenwärmerrahmen nach einem der Ansprüche 1 bis 3, wobei der erste gekrümmte Bereich (232) des Bandes eine Reihe von Erhebungen an dessen innerer Fläche aufweist, die mit dem Überlappungsbereich im Eingriff sind, der durch den zweiten Bandbereich gebildet wird, um einen Widerstand für die relative Gleitbewegung der beiden Bandbereiche (232, 234) zu bilden, wenn eine Einstellung der Gesamtlänge des Bandes erfolgt.

5. Ohrenwärmerrahmen nach Anspruch 1, der über den Ohren einer Person zu tragen ist und sich über den Hinterkopf der Person erstreckt, mit:
zwei Ohrrnuschelaufnahmen (20), wobei jede Ohrmuschelaufnahme an gegenüberliegenden Endbereichen des Bandes, befestigt ist, wobei die Befestigung durch einen Befestigungsflansch (22) gebildet ist, wobei jede Ohrmuschelaufnahme (20) einen Aufnahmebereich (24) mit einer zentralen Öffnung darin aufweist, wobei die Krümmung eines mittleren Bereichs (32) des Bandes und der Befestigungsflansch (22) mit dem Aufnahmebereich (24) die Ohrmuschelaufnahmen (20) von den gegenüberliegenden Enden des Bandes (18) nach innen aufeinander zu führen; und
einer Gewebeeinrichtung (40), die das Band (18) und die Ohrmuschelaufnahmen (20) auf beiden Seiten bedeckt.

6. Ohrenwärmerrahmen nach Anspruch 5, wobei der Aufnahmebereich eine kegelstumpf-artige Form aufweist.

7. Ohrenwärmerrahmen nach Anspruch 5 oder 6, wobei der Befestigungsflansch und der Aufnahmebereich als Einheit und schräg zueinander ausgebildet sind.

8. Ohrenwärmerrahmen nach einem der Ansprüche 5 bis 7, wobei die Gewebeeinrichtung eine Tasche in dem Aufnahmebereich der Ohrmuschelaufnahmen bildet.

9. Ohrenwärmerrahmen nach Anspruch 5, wobei die beiden Ohrmuschelaufnahmen an den Enden des ersten gekrümmten Bereichs gegenüberliegend zu deren Überlappungsbereich angebracht sind, wobei die Befestigung der Ohrmuschelaufnahme eine Drehung des Aufnahmebereichs um die Befestigung herum ermöglicht.

10. Ohrenwärmerrahmen nach Anspruch 9, wobei die Gewebeeinrichtung das Band und die Ohrmuschelaufnahmen einhüllt.

11. Ohrwärmerrahmen nach einen der Ansprüche 9 oder 10, wobei der Aufnahmebereich eine kegelstumpf-artige Form aufweist.

12. Ohrenwärmerrahmen nach Anspruch 5, wobei
die zwei Ohrmuschelaufnahmen an dem freien Ende des ersten gekrümmten Bereichs bzw. dem freien Ende des zweiten gekrümmten Bereichs angebracht sind, wobei die Ohrmuschelaufnahmen einen. Hohlraum, der von zwei Halteflanschen gebildet wird, und einen halbkreisförmigen Rahmenbereich definieren, der sich zwischen gegenüberliegenden. Erhebungen der Halteflansche erstreckt, wobei der halbkreisförmige Bereich und die Halteflansche eine zentrale Öffnung definieren, wobei der Befestigungsflansch von den Halteflanschen in einer Richtung entgegengesetzt zu dem halbkreisförmigen Bereich hervorragt, und wobei der Befestigungsflansch eine Einrichtung daran aufweist, um die Ohrmuschelaufnahmen drehbar an den freien Enden des ersten und des zweiten gekrümmten Bereichs anzubringen.

13. Ohrenwärmerrahmen nach Anspruch 12, wobei die zwei Halteflansche in einer V-Form ausgebildet sind und wobei der Befestigungsflansch aus dem Verbindungsbereich der beiden Halteflansche hervorragt.

14. Ohrenwärmerrahmen nach einem der Ansprüche 12 oder 13, wobei die Gewebeeinrichtung eine Tasche in dem Hohlraum der Ohrmuschelaufnahmen bildet.

15. Ohrenwärmerrahmen nach einem der Ansprüche 12 bis 14, wobei die Gewebeeinrichtung aus mehreren Gewebestücken aufgebaut ist.

16. Ohrenwärmerrahmen nach einem der Ansprüche 12 bis 15, wobei die beiden Ohrmuschelaufnahmen drehbar in eine Position an den freien Enden des ersten und des zweiten gekrümmten Bereichs so eingreifen, dass die Ohrmuschelaufnahmen zu dem Band ausgerichtet sind.

17. Ohrenwärmerrahmen nach einem der Ansprüche 12 bis 16, wobei:
ein Abschnitt benachbart zu dem Ende des ersten gekrümmten Bereichs des Bandes weniger breit als das Ende des ersten gekrümmten Bereichs des Bandes ist, und
ein Abschnitt benachbart zu dem Ende des zweiten gekrümmten Bereichs des Bandes weniger breit als das Ende des zweiten gekrümmten Bereichs des Bandes ist.

18. Ohrenwärmerrahmen nach einem der Ansprüche 12 bis 17, wobei das Band und die beiden Halteflansche die Ohrenmuschelaüfnahmen nach innen aufeinander zu führen, um die Position des Ohrenwärmerrahmens beizubehalten.

19. Ohrenwärmerrahmen nach einem der Ansprüche 12 bis 18, wobei der Ohrenwärmerrahmen die Position um die Rückseite des Kopfes der Person oder um den Nacken beibehält, ohne dass eine Halterung auf der Oberseite des Kopfes der Person vorgesehen ist.

20. Ohrenwärmerrahmen nach Anspruch 5, wobei das Band flexibel ist und mindestens ein Teil der Ohrmuschelaufnahme eine kegelstumpf-artige Konfiguration und eine zentrale Öffnung aufweist.

21. Ohrenwärmerrahmen nach Anspruch 20, wobei die Gewebeeinrichtung und die Ohrmuschelaufnahmen eine Tasche bilden, wodurch eine Öffnung in der Gewebeeinrichtung zum Einführen eines Ohrhörers in die Tasche gebildet wird.

22. Ohrenwärmerrahmen nach einem der Ansprüche 20 oder 21, wobei das flexible Band eine Innenseite und eine Außenseite aufweist, wobei jede Ohrmuschelaufnahme eine Innenseite u nd e ine A ußenseite aufweist u nd wobei d ie G ewebeeinrichtung die Innenseite und die Außenseite des flexiblen Bandes und die Innenseite und die Außenseite jeder Ohrmuschelaufnahme bedeckt.

23. Ohrenwärmerrahmen nach einem der Ansprüche 20 bis 23, wobei die Ohrmuschelaufnahmen mittels Scharnieren mit den Endbereichen des Bandes verbunden sind.

24. Ohrenwärmerrahmen nach Anspruch 23, wobei die Scharnierverbindung zwischen den Endbereichen und dem Band durch ein flexibles Scharnier gebildet ist.

## Revendications

1. Cadre de réchauffement pour oreilles (210), comprenant :
une bande (230) comportant :
une première partie courbe (232) comportant une partie agrandie (246) située à une première extrémité (226) et un passage (238) situé à une seconde extrémité, et
une seconde partie courbe (234) comportant une partie agrandie (248) à située à une première extrémité (228) et un passage (236) situé à une seconde extrémité, chaque passage (236, 238) comportant une première partie fendue permettant le coulissement au travers de celle-ci de la partie courbe opposée de ladite bande (230) et comportant une seconde partie fendue permettant le coulissement au travers de celle-ci de la partie agrandie (246, 248) de la partie courbe opposée de ladite bande, ce qui permet à une extrémité de chacune des première et seconde parties courbes (232, 234) de se recouvrir et d'être fixée en coulissement sur une partie courbe opposée, de sorte qu'il est possible de régler la longueur totale relative de la bande (230).

2. Cadre de réchauffement pour oreilles selon la revendication 1, comprenant en outre :
deux caches-oreille dont chacun comporte une tête de fixation, chaque tête de fixation étant fixée sur une partie agrandie d'une partie courbe de ladite bande.

3. Cadre de réchauffement pour oreilles selon l'une quelconque des revendications 1 ou 2, dans lequel le passage de la première partie courbe de ladite bande est formé intégralement avec la première partie courbe de ladite bande, le passage de la seconde partie courbe de ladite bande est formé intégralement avec la seconde partie courbe de ladite bande.

4. Cadre de réchauffement pour oreilles selon l'une quelconque des revendications 1 à 3, dans lequel la première partie courbe de la bande comprend une série de rugosités en élévation situées sur la surface interne de celle-ci et qui s'engagent avec la partie de recouvrement formée par la seconde partie de bande afin de former une résistance au mouvement de glissement relatif des deux parties de bande, lorsque le réglage de la longueur totale de la bande est souhaité.

5. Cadre de réchauffement pour oreilles selon la revendication 1, adapté pour être supporté par les oreilles d'un individu et s'étendant autour du cou ou de l'arrière de la tête d'un individu, comprenant :
deux caches-oreille (20), chaque cache-oreille étant fixé aux parties d'extrémité opposées de la bande, la fixation étant formée par une bride de fixation (22), chaque cache-oreille (20) comprenant une partie de cache (24) comprenant dans celle-ci une ouverture centrale (34), la courbure d'une partie centrale (32) de la bande et la bride de fixation (22) comportant la partie de cache (24) dirigeant les caches-oreille (20) vers l'intérieur vers l'une ou l'autre des extrémités opposées de la bande (18) ; et
des moyens de tissu (40) qui recouvrent la bande (18) et les cache-oreille (20) sur les deux côtés.

6. Cadre de réchauffement pour oreilles selon la revendication 5, dans lequel la partie de cache a une forme de cône tronqué.

7. Cadre de réchauffement pour oreilles selon l'une quelconque des revendications 5 ou 6, dans lequel la bride de fixation est intégralement formée avec la partie de cache selon un angle oblique par rapport à celle-ci.

8. Cadre de réchauffement pour oreilles selon l'une quelconque des revendications 5 à 7, dans lequel les moyens de tissu forment une poche à l'intérieur de la partie de cache des caches-oreille.

9. Dispositif de cadre de réchauffement pour oreilles selon la revendication 5, dans lequel :
lesdits deux caches-oreille sont fixés sur les extrémités de la première partie courbe et de la seconde partie courbe respectivement en opposition à leur recouvrement, la fixation du cache-oreille sur la bande permettant la rotation de la partie de cache autour de la fixation.

10. Cadre de réchauffement pour oreilles selon la revendication 9, dans lequel les moyens de tissu enveloppent la bande et les caches-oreille.

11. Cadre de réchauffement pour oreilles selon l'une quelconque des revendications 9 ou 10, dans lequel la partie de cache a une forme de cône tronqué.

12. Cadre de réchauffement pour oreilles selon la revendication 5, dans lequel :
lesdits deux caches-oreille sont fixés respectivement sur l'extrémité libre de la première partie courbe et sur l'extrémité de la seconde partie courbe, les caches-oreille définissant une cavité formée à partir de deux brides de support, et une partie de cadre semi-circulaire qui s'étend entre des saillies en opposition des brides de support, la partie-semi-circulaire et les brides de support définissant une ouverture centrale, la bride de fixation étant en saillie à partir des brides de support dans une direction opposée à la partie semi-circulaire, la bride de fixation comprenant sur elle des moyens destinés à fixer en rotation les caches-oreille sur les extrémités libres des première et seconde parties courbes.

13. Cadre de réchauffement pour oreilles selon la revendication 12, dans lequel lesdites deux brides de support ont une forme en V, et la bride de fixation est en saillie à partir du sommet des brides de support.

14. Cadre de réchauttement pour oreilles selon l'une quelconque des revendications 12 ou 13, dans lequel les moyens de tissu forment une poche à l'intérieur de la cavité des caches-oreille.

15. Cadre de réchauffement pour oreilles selon l'une quelconque des revendications 12 à 14, dans lequel les moyens de tissu sont fabriqués à partir d'une pluralité de pièces de tissu.

16. Cadre de réchauffement pour oreilles selon l'une quelconque des revendications 12 à 15, dans lequel les deux caches-oreille s'engagent en position sur les extrémités libres des première et seconde parties courbes de sorte que les caches-oreille sont en alignement avec la bande.

17. Cadre de réchauffement pour oreilles selon l'une quelconque des revendications 12 à 16, dans lequel :
une section située à proximité de l'extrémité de la première partie courbe de la bande est moins large que l'extrémité de la première partie courbe de la bande, et
une section située à proximité de l'extrémité de la seconde partie courbe de la bande est moins large que l'extrémité de la seconde partie courbe de la bande.

18. Cadre de réchauffement pour oreilles selon l'une quelconque des revendications 12 à 17, dans lequel la bande et les deux brides de support dirigent les caches-oreille vers l'intérieur l'un vers l'autre afin de maintenir en place le dispositif de protection.

19. Cadre de réchauffement pour oreilles selon l'une quelconque des revendications 12 à 18, dans lequel le dispositif de protection reste en position autour de l'arrière de la tête ou du cou de l'individu sans nécessiter de support autour du sommet de la tête de l'individu.

20. Cadre de réchauffement pour oreilles selon la revendication 5, dans lequel ladite bande est flexible et au moins une partie dudit cache-oreille a une configuration en cône tronqué et comporte une ouverture centrale.

21. Cadre de réchauffement pour oreilles selon la revendication 20, dans lequel les moyens de tissu et les caches-oreille forment une poche, ce qui permet de former des moyens d'ouverture dans les moyens de tissu afin d'introduire un écouteur à l'intérieur de la poche.

22. Cadre de réchauffement pour oreilles selon l'une quelconque des revendications 20 ou 21, dans lequel la bande flexible comprend un côté interne et un côté externe, chacun des caches-oreille comprend un côté interne et un côté externe, et les moyens de tissu recouvrent à la fois le côté interne et le côté externe de la bande flexible et recouvrent à la fois le côté interne et le côté externe de chaque cache-oreille.

23. Cadre de réchauffement pour oreilles selon l'une quelconque des revendications 20 à 23, dans lequel les caches-oreille sont connectés en articulation aux parties d'extrémité de la bande.

24. Cadre de réchauffement pour oreilles selon la revendication 23, dans lequel la connexion en articulation entre les parties d'extrémité et la bande est formée par une articulation active.
